# EUROPEAN PATENT SPECIFICATION

(11) **EP 4 081 139 B1**
(45) Date of publication and mention of the grant of the patent: **21.02.2024**
(21) Application number: 20829659.0
(22) Date of filing: 06.12.2020
(51) Int. Cl.: A61B 17/34, A61B 90/00, A61B 34/20, A61B 90/11

(54) **TROCAR WITH MOVABLE CAMERA AND BUILT-IN POSITION SENSOR**
TROKAR MIT BEWEGLICHER KAMERA UND EINGEBAUTEM POSITIONSSENSOR
TROCART AVEC CAMÉRA MOBILE ET CAPTEUR DE POSITION INTÉGRÉ

(30) Priority: 29.12.2019 US 201916729435
(43) Date of publication of application: 02.11.2022
(73) Proprietor: Biosense Webster (Israel) Ltd., Yokneam 2066717 (IL)
(72) Inventor: ALGAWI, Yehuda, 2066717 Yokneam (IL); GOVARI, Assaf, 2066717 Yokneam (IL); SITNITSKY, Ilya, 2066717 Yokneam (IL); KATZIR, Stanislav, 2066717 Yokneam (IL)
(74) Representative: Carpmaels & Ransford LLP
(86) International application number: PCT/IB2020/061560
(87) International publication number: WO 2021/137055

(56) References cited:
- US-A- 5 305 121
- US-A1- 2015 141 755
- US-A1- 2016 128 722
- US-A1- 2019 350 619

## Description

### FIELD OF THE INVENTION

The present invention is defined by the appended claims and relates generally to invasive medical tools, and particularly to invasive medical tools incorporating a camera.

### BACKGROUND OF THE INVENTION

Techniques for image-guided probing of an organ of a patient were previously proposed in the patent literature. For example, U.S. Patent Application Publication 2011/0160535 describes a disposable access port for use in endoscopic procedures, including laparoscopic procedures. The access port includes a cannula with an embedded camera in communication with an external control box. In operation, a trocar is combined with the access port to facilitate insertion of the access port into an anatomical site. Prior to insertion, the camera is pushed inside the cannula, where it remains during insertion. The trocar is removed after the access port has been inserted to allow surgical instruments to access the anatomical site. During removal of the trocar, a portion of the trocar urges the camera out of the cannula, thereby allowing visualization of the anatomical site. The camera can be fixedly or adjustably mounted on the port. A camera may also be mounted on the trocar. The trocar may include irrigation and suction channels to facilitate a clear view of the anatomical site.

As another example, U.S. Patent Application Publication 2013/0282041 describes a viewing trocar assembly including a tubular body having a proximal end and a distal end, and an opening provided at the distal end, and at least one imaging device positioned on an outer wall of the distal end of the tubular body, wherein the at least one imaging device is adjacent to the outer wall of the distal end of the tubular body when in an inactivated position, and wherein the at least one imaging device is extended further away from the outer wall of the distal end of the tubular body when in an activated position than when in the inactivated position. The disclosed imaging device is maneuverable once positioned inside a patient's body, thus providing improved imaging capabilities.

U.S. Patent Application Publication 2015/0141755 describes a tubular retractor and an imaging insert including an illumination assembly and a camera module. The tubular retractor is sized and configured to receive an insert therein, which includes an illumination assembly and a camera module. In an embodiment, the camera module is slidably received within the insert, allowing for the camera module to be raised and lowered with respect to the insert. In an embodiment, an upper ring on the top of the proximal head of the insert includes a plurality of slots configured to receive flex cables therein. In an embodiment, two flex cables are provided, each directed to a different one of the slots. Associated with each slot is a rotatable knob which, when actuated, causes the flex cable to be fed into or out of the insert. With actuation of the rotary knob, the associated flex cable can be raised or lowered within the insert, nearer or further from the distal end. By lowering or raising the flex cables, the cameras disposed on the distal ends thereof are moved closer or further from the distal end.

### SUMMARY OF THE INVENTION

The present invention provides a trocar, according to appended claim 1.

In some embodiments, the movable element is configured to be slid in a channel formed at an inner wall of a cannula, parallel to the longitudinal axis, and wherein a distal end of the movable element is coupled to the camera.

In some embodiments, the trocar further includes a slide control handle coupled to a proximal end of the movable element, the slide control handle including a rotatable camera-position control knob configured to be rotated and, by being rotated, to move the movable element and the camera.

The trocar further includes a position sensor, which is attached to an inner wall of a distal end of the cannula, and is configured to generate signals indicative of a position of the distal end in the organ.

In an embodiment, the position sensor is a magnetic position sensor. In another embodiment, the camera is tilted relative to the longitudinal axis, so as to have a viewing direction that captures a distal opening of the cannula.

There is additionally provided, in accordance with another embodiment of the present invention, an apparatus including a trocar and a slide control handle.

There is further provided, in accordance with another embodiment of the present invention, a system including a trocar and a processor.

There is furthermore described, a method not defined by the invention, the method including inserting a trocar into an organ of a patient, the trocar including a cannula having a longitudinal axis, a camera fitted inside the cannula, and a movable element, which is coupled to the camera and is configured to be moved along the longitudinal axis of the cannula and to move the camera along the cannula. The slide control handle coupled to a proximal end of the movable element and configured to move the movable element and the camera. The movable element and the camera are moved using a slide control handle coupled to a proximal end of the movable element.

There is further yet described, a method not defined by the invention, the method including inserting a trocar into an organ of a patient, the trocar including a cannula having a longitudinal axis, a camera fitted inside the cannula, a movable element, which is coupled to the camera and is configured to be moved along the longitudinal axis of the cannula and to move the camera along the cannula, and a position sensor, which is attached to an inner wall of a distal end of the cannula without obstructing a field of view of the camera, and is configured to generate signals indicative of a position of the distal end in the organ. Using the signals generated by the position sensor, the position of the distal end of the trocar in the organ is estimated.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a brain procedure using a surgical apparatus comprising a trocar comprising a slidable camera and a position sensor, in accordance with an embodiment of the present invention;
Fig. 2 is a schematic, pictorial illustration of the trocar applied in the brain procedure of Fig. 1, in accordance with an embodiment of the present invention; and
Fig. 3 is a flow chart that schematically illustrates a method and algorithm for achieving a best visual image from slidable camera of Fig. 1 and registering the image with a reference medical image.

### DETAILED DESCRIPTION OF EMBODIMENTS

### OVERVIEW

Some invasive medical procedures require a way to visually guide a medical probe to an organ, such as a brain, of a patient. In some invasive procedures, to insert a medical probe or other tool into the body of a patient, a trocar, which serves as a penetrating portal, is first placed in an entry location. In addition to being a portal for the probe, the trocar, which comprises a cannula, may be used for irrigation and to drain bodily fluids, as well as other fluids. Moreover, the trocar may be equipped with a camera to assist in the visual navigation of the probe to target tissue.

For example, brain procedures may require navigating a distal end of a probe inserted into the brain via a hole made in the skull. For the procedure, a trocar with a camera may be inserted to enable a physician to acquire images of a target brain tissue, and a treating probe is advanced via the trocar and visually guided to treat the target brain tissue, for example, an infected or bleeding brain tissue.

However, if the camera of the trocar is located too far distally inside the trocar, the acquired image is obscured by blood or other matter; if the camera is located too proximally inside the trocar the acquired image is of a poor quality and may show the trocar wall rather than the desired view.

Embodiments of the present invention that are described hereinafter provide a trocar that has, fitted internally to a wall of the cannula, a movable camera and a moving mechanism to move the camera parallel to a longitudinal axis of the cannula (i.e., over a range between proximal and distal positions), to a location inside the cannula from which the camera can provide a best view, such as of target tissue and/or of a treating probe.

In some embodiments, a sliding movement of the camera is provided using a channel within the cannula, within which the camera can slide using a movable element in the channel to which the camera is coupled. In some embodiments, the movable element is a flexible tube that is coupled to the camera via a sliding adapter on which the camera is mounted. The flexible tube further contains camera wiring. The flexible tube, also called hereinafter "a camera control guide," connects the camera to an external control that is configured to cause the control guide to slide. The external control is typically fitted with an a slide control handle coupled to a proximal end of the movable element, the slide control handle including a rotatable camera-position control knob configured to be rotated and, by being rotated, to move the movable element and the camera. For example, after the trocar has been inserted, the physician can rotate the camera-position control knob to slide the movable element along its channel, which causes the camera to slide as described above. The physician is thus able to position the camera such that the camera is not obscured and can provide a good image.

In other embodiments, other types of movable elements may be used to move the camera, such as elastic elements (e.g., a spring), a piston, or shape-memory-based mechanical elements. In yet another embodiment, the movement may be realized using, for example, an electrical motor coupled to the camera to move on a rail fitted inside the cannula. Similarly, other kinds of external controls may be realized, such as a slider button or an electrical switch.

According to the invention, a position sensor is attached to the internal wall of the cannula of the trocar at a distal end of the cannula. Sensor wiring, providing location data from the sensor, is passed from the sensor, through the camera control guide, and to a processor that provides the physician with location data for the trocar distal end to, for example, register a captured image from the movable (e.g., slidable) camera with a reference medical image (e.g., an MRI image).

By optimizing visual image acquisition using a position-adjustable (i.e., movable) camera of a trocar, the disclosed technique may enable improved outcomes of minimally invasive medical procedures.

### SYSTEM DESCRIPTION

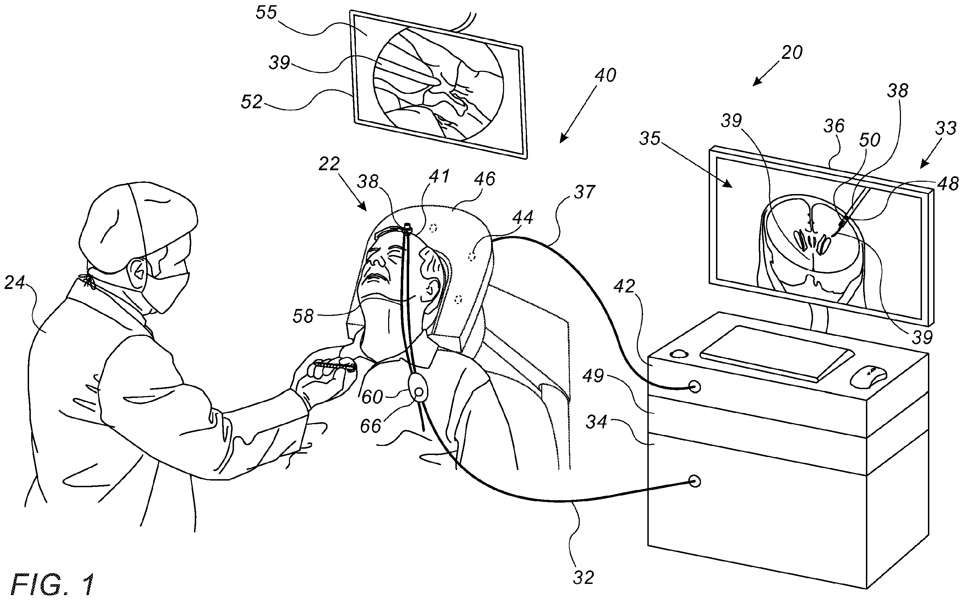

Fig. 1 is a schematic, pictorial illustration of a brain procedure using a surgical apparatus 28 comprising a trocar 38 comprising a slidable camera 48 and a position sensor 50, in accordance with an embodiment of the present invention. In some embodiments, a brain diagnostics and treatment system 20, which comprises surgical apparatus 28, is configured to carry out a brain procedure, such as treating an infection from brain tissue of a patient 22. In the shown embodiment, trocar 38 is used to penetrate the skull so that a physician 24 can insert a probe 39 into a head 41 of patient 22 to access brain tissue. Subsequently, probe 39 may be operated using the trocar-attached slidable camera 48 and magnetic position sensor 50. Typically, treating probe 39 may be operated by a second physician (not shown).

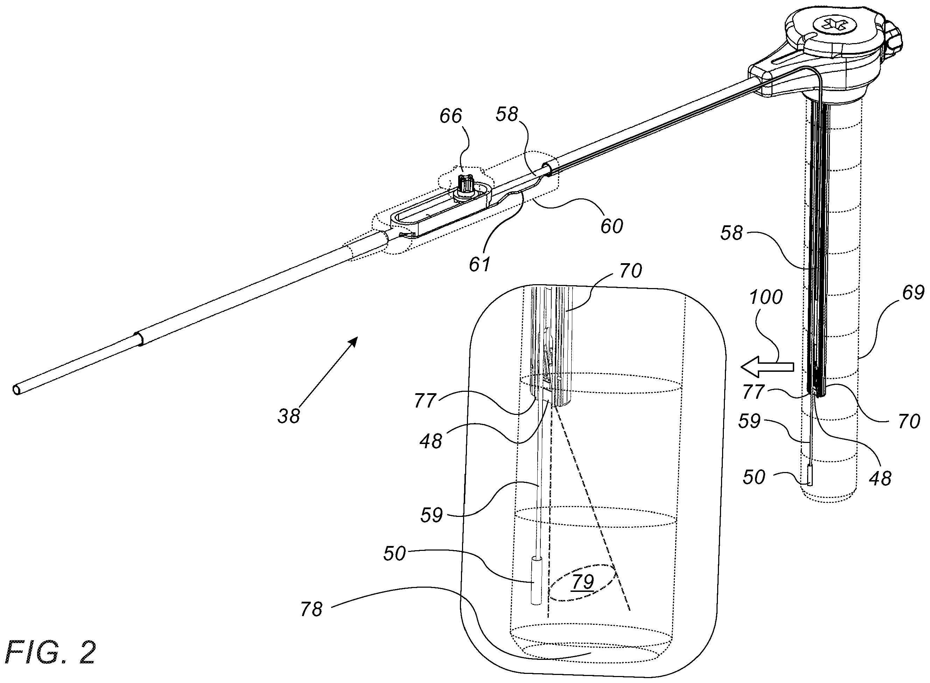

In the shown embodiment, a slide control handle 60 includes a rotatable camera-position control knob 66 located on a slide control to move a camera control guide 58 (i.e., movable element 58). Guide 58 enters a proximal end of trocar 38 and is coupled on its distal end to camera 48. By rotating knob 66, physician 24 can slide camera 48 inside trocar 38 to adjust a position of slidable camera 48 for a best view, as further described in Fig. 2.

Slide control handle 60 may further include wiring to sensor 50 and may include additional control elements to assist physician 24 to perform the procedure, such as command buttons to capture an image from camera 48 and to register the image with a reference medical image.

System 20 comprises a magnetic position-tracking system, which is configured to track a position of sensor 50 in the brain. The magnetic position-tracking system comprises a location pad 40, which comprises field generators 44 fixed on a frame 46. In the exemplary configuration shown in Fig. 1, pad 40 comprises five field generators 44, but may alternatively comprise any other suitable number of generators 44. Pad 40 further comprises a pillow (not shown) placed under head 41 of patient 22, such that generators 44 are located at fixed, known positions external to head 41. The position sensor generates position signals in response to sensing external magnetic fields generated by field generators 44, thereby enabling a processor 34 to estimate the position of sensor 50 and therefore a position of a distal edge of trocar 38 inside the head of patient 22.

This technique of position sensing is implemented in various medical applications, for example, in the CARTO^{™} system, produced by Biosense Webster Inc. (Irvine, CA) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

In some embodiments, system 20 comprises a console 33, which comprises a memory 49, and a driver circuit 42 configured to drive field generators 44, via a cable 37, with suitable signals so as to generate magnetic fields in a predefined working volume in space around head 41.

Processor 34 is typically a general-purpose computer, with suitable front end and interface circuits for receiving images from camera 48 and signals from position sensor 50 via a cable 32, and for controlling other components of system 20 described herein.

In some embodiments, processor 34 is configured to register an image produced by camera 48 with a medical image, such as an MRI image. Processor 34 may further register the position of the distal end that is estimated using position sensor 50. Processor 34 is able to register a visual image by estimating a position of a distal edge of trocar 38 using position sensor 50. Processor 34 is configured to register the camera image and the reference medical image in the coordinate system of the magnetic position-tracking system and/or in a coordinate system of the reference medical image.

In some embodiments, system 20 comprises a video display 52 that shows an image 55 taken by sliding camera 48. In the shown image, a distal end of treating probe 39 can be seen engaging brain tissue.

In some embodiments, processor 34 is configured to receive, via an interface (not shown), one or more anatomical images, such as reference MRI images depicting two-dimensional (2D) slices of head 41. Processor 34 is configured to select one or more slices from the MRI images, register it with a real-time camera image, such as image 55, to produce a combined image, such as an image 35, and display the selected combined slice to physician 24 on user display 36. In the example of Fig. 1, combined image 35 depicts a sectional coronal view of anterior brain tissue of patient 22.

Console 33 further comprises input devices, such as a keyboard and a mouse, for controlling the operation of the console, and a user display 36, which is configured to display the data (e.g., images) received from processor 34 and/or to display inputs inserted by a user using the input devices (e.g., by physician 24).

Fig. 1 shows only elements related to the disclosed techniques for the sake of simplicity and clarity. System 20 typically comprises additional or alternative modules and elements that are not directly related to the disclosed techniques, and thus are intentionally omitted from Fig. 1 and from the corresponding description. Alternative embodiments are possible to move the camera, such as using a slider button on handle 60 instead of a rotatable knob.

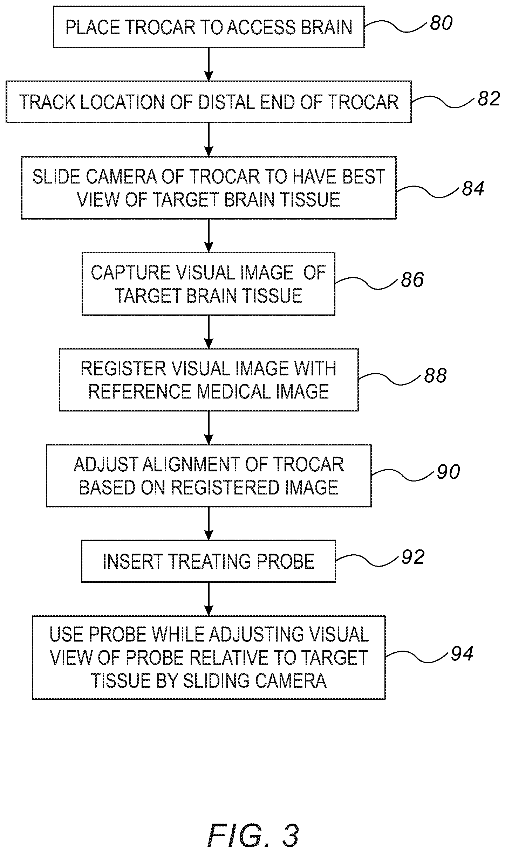

Processor 34 may be programmed in software to carry out the functions that are used by the system, and to store data in memory 49 to be processed or otherwise used by the software. The software may be downloaded to the processor in electronic form, over a network, for example, or it may be provided on non-transitory tangible media, such as optical, magnetic or electronic memory media. Alternatively, some or all of the functions of processor 34 may be carried out by dedicated or programmable digital hardware components. In particular, processor 34 runs a dedicated algorithm as disclosed herein, including in Fig. 3, that enables processor 34 to perform the disclosed steps, as further described below.

### TROCAR WITH SLIDABLE CAMERA AND BUILT-IN POSITION SENSOR

Fig. 2 is a schematic, pictorial illustration of the trocar 38 applied in the brain procedure of Fig. 1, in accordance with an embodiment of the present invention. As seen, trocar 38 includes a channel 70 inside cannula 69, which provides a track in which to slide camera control guide 58. Camera 48 is coupled to guide 58, for example, via a sliding adapter 77 on which the camera is mounted, so that the camera can slide distally (e.g., down) or proximally (e.g., up) inside cannula 69.

To slide camera control guide 58 distally, the physician rotates control knob 66 on external control handle 60 in a first direction. The rotation of knob 66 moves camera control guide 58 forward, which is translated (e.g., by the approximately right angle turning of flexible guide 58) into distal motion of camera 48 inside cannula 69. By rotating knob 66 in the other direction, the physician pulls camera control guide 58 backwards, which is translated into proximal motion of camera 48 in cannula 69.

As further seen, magnetic sensor 50 is fixed to an inner wall of cannula 69, and its wiring 59 is routed (61) with the wiring of camera 48 to control handle 60, from which the wiring is routed to the console via cable 32 of Fig. 1.

A zoom-in (100) on a distal end of cannula 69 shows that camera 48 is mounted in a tilted configuration so as to have a central distal viewing direction pointing at a center of a distal opening 78 of cannula 69. At the same time, sensor 50 is sufficiently thin to be attached to the cannula wall, and therefore does not obstruct the field of view 79 of camera 48.

The configuration of trocar 38 in Fig. 2 is depicted by way of example for the sake of conceptual clarity. In other embodiments, additional elements may be included, such as additional ports in trocar 38 to insert medical tools to the target brain location.

Fig. 3 is a flow chart that schematically illustrates a method and algorithm for achieving a best visual image from slidable camera 48 of Fig. 1 and registering the image with a reference medical image, in accordance with an embodiment of the present disclosure.

The process begins when physician 24 places trocar 38 to access the brain, at a trocar placement step 80.

Next, physician 24 operates system 20 to magnetically track, using signals from sensor 50, a location in the brain of a distal end of trocar 38, at a trocar position tracking step 82. Next, physician 24 operates apparatus 28, by rotating control knob 66, to slide camera 48 to have a best view (e.g., on display 52) of target brain tissue, at camera position adjustment step 84. In an image capturing step 86, physician 24 captures an image by camera 48, to register with a reference medical image.

At an image registration step 88, based on the tracked position of trocar's 38 distal end (using sensor 50), processor 34 registers the captured image (by camera 48) with a respective reference medical image stored in memory 49, such as from an MRI scan, to produce combined image 35. In an embodiment, processor 34 is further configured to correct the reference medical images based on the registered images, for example, if the treatment removes brain tissue. In another embodiment, the processor is further configured to alert a user to a detected discrepancy between the visual image and the reference image due to, for example, a larger tumor size detected by camera 48 because of tumor growth since the reference image was taken.

Next, at a trocar adjustment step 90, using combined image 35, physician 24 adjusts an alignment of trocar 38, e.g., to allow best access to target brain tissue, such as an infected tissue. Physician 24 then inserts a treating probe 39, at a probe insertion step 92. Finally, at a treating step 94, physician 24 uses probe 39 to treat target tissue under visual guidance provided by slidable camera 48, whose position is adjusted by physician 24 by using knob 66 to obtain a best view of probe 39 relative to target tissue.

The example flow chart shown in Fig. 3 is chosen purely for the sake of conceptual clarity. In alternative embodiments physician 24 may perform additional steps, such as employing additional monitoring steps (e.g., fluoroscopy) to verify the successful outcome of the procedure, and/or apply irrigation to clear view for slidable camera 48.

Although the embodiments described herein mainly address brain procedures, the methods and systems described herein can also be used in other applications that require guiding a medical device in other organs, such as located in the abdomen or the chest.

It will thus be appreciated that the embodiments described above are cited by way of example, and that the present invention is not limited to what has been particularly shown and described hereinabove. Rather, the scope of the present invention is defined by the appended claims.

## Claims

1. A trocar (38) for insertion into an organ of a patient, the trocar comprising:
a cannula (69) having a longitudinal axis;
a camera (48) fitted inside the cannula;
a movable element (58), which is coupled to the camera and is configured to be moved along the longitudinal axis of the cannula and to move the camera along the cannula; and **characterised by**
a position sensor (50), which is attached to an inner wall of a distal end of the cannula, and is configured to generate signals indicative of a position of the distal end in the organ, wherein sensor wiring (59), providing location data from the position sensor, is passed from the position sensor through the movable element.

2. The trocar according to claim 1, wherein the movable element is configured to be slid in a channel (70) formed at an inner wall of a cannula, parallel to the longitudinal axis, and wherein a distal end of the movable element is coupled to the camera.

3. The trocar according to claim 2, and comprising a slide control handle (60) coupled to a proximal end of the movable element, the slide control handle comprising a rotatable camera-position control knob (66) configured to be rotated and, by being rotated, to move the movable element and the camera.

4. The trocar according to any preceding claim, wherein the position sensor is a magnetic position sensor.

5. The trocar according to any preceding claim, wherein the camera is tilted relative to the longitudinal axis, so as to have a viewing direction that captures a distal opening (78) of the cannula.

6. An apparatus (28), comprising:
the trocar of claim 1; and
a slide control handle coupled to a proximal end of the movable element and configured to move the movable element and the camera.

7. The apparatus according to claim 6, wherein the movable element is configured to be slid in a channel formed at an inner wall of a cannula, parallel to the longitudinal axis, wherein a distal end of the movable element is coupled to the camera, and wherein the slide control handle comprises a rotatable camera-position control knob configured to be rotated and, by being rotated, slide the movable element.

8. A system, comprising:
the trocar of any of claims 1 to 5; and
a processor, which is configured to:
using the signals generated by the position sensor, estimate the position of the distal end of the trocar in the organ.

9. The system according to claim 8, wherein the processor is further configured to:
based on the estimated position, register an image acquired by the camera with a reference medical image; and
present the image acquired by the camera and the reference medical image, registered with one another, to a user.

## Patentansprüche

1. Trokar (38) zum Einführen in ein Organ eines Patienten, wobei der Trokar Folgendes umfasst:
eine Kanüle (69) mit einer Längsachse,
eine in der Kanüle angebrachte Kamera (48),
ein bewegliches Element (58), das an die Kamera gekoppelt und dazu ausgestaltet ist, entlang der Längsachse der Kanüle bewegt zu werden und die Kamera entlang der Kanüle zu bewegen, und **gekennzeichnet durch**
einen Positionssensor (50), der an einer Innenwand eines distalen Endes der Kanüle angebracht und dazu ausgestaltet ist, Signale zu erzeugen, die eine Position des distalen Endes in dem Organ angeben, wobei Sensordrähte (59), die Ortsdaten von dem Positionssensor bereitstellen, von dem Positionssensor durch das bewegliche Element gehen.

2. Trokar nach Anspruch 1, wobei das bewegliche Element dazu ausgestaltet ist, in einem an einer Innenwand einer Kanüle gebildeten Kanal (70) parallel zu der Längsachse verschoben zu werden, und wobei ein distales Ende des beweglichen Elements an die Kamera gekoppelt ist.

3. Trokar nach Anspruch 2, umfassend einen Verschiebungssteuergriff (60), der an ein proximales Ende des beweglichen Elements gekoppelt ist, wobei der Verschiebungssteuergriff einen drehbaren Kamerapositionssteuerknopf (66) umfasst, der dazu ausgestaltet ist, gedreht zu werden und, indem er gedreht wird, das bewegliche Element und die Kamera zu bewegen.

4. Trokar nach einem der vorhergehenden Ansprüche, wobei der Positionssensor ein magnetischer Positionssensor ist.

5. Trokar nach einem der vorhergehenden Ansprüche, wobei die Kamera bezüglich der Längsachse gekippt ist, so dass sie über eine Blickrichtung verfügt, die eine distale Öffnung (78) der Kanüle erfasst.

6. Vorrichtung (28), umfassend:
den Trokar nach Anspruch 1 und
einen Verschiebungssteuergriff, der an ein proximales Ende des beweglichen Elements gekoppelt und dazu ausgestaltet ist, das bewegliche Element und die Kamera zu bewegen.

7. Vorrichtung nach Anspruch 6, wobei das bewegliche Element dazu ausgestaltet ist, in einem an einer Innenwand einer Kanüle gebildeten Kanal parallel zu der Längsachse verschoben zu werden, und wobei ein distales Ende des beweglichen Elements an die Kamera gekoppelt ist und wobei der Verschiebungssteuergriff einen drehbaren Kamerapositionssteuerknopf umfasst, der dazu ausgestaltet ist, gedreht zu werden und, indem er gedreht wird, das bewegliche Element zu verschieben.

8. System, umfassend:
den Trokar nach einem der Ansprüche 1 bis 5 und
einen Prozessor, der dazu ausgestaltet ist,
unter Verwendung der von dem Positionssensor erzeugten Signale die Position des distalen Endes des Trokars in dem Organ zu schätzen.

9. System nach Anspruch 8, wobei der Prozessor ferner dazu ausgestaltet ist,
ein von der Kamera aufgenommenes Bild auf der Grundlage der geschätzten Position einem medizinischen Bezugsbild zuordnen, und
das von der Kamera aufgenommene Bild und das medizinische Bezugsbild, die einander zugeordnet worden sind, einem Benutzer vorzulegen.

## Revendications

1. Trocart (38) destiné à être inséré dans un organe d'un patient, le trocart comprenant :
une canule (69) ayant un axe longitudinal ;
une caméra (48) montée à l'intérieur de la canule ;
un élément mobile (58), qui est couplé à la caméra et est configuré pour être déplacé le long de l'axe longitudinal de la canule et pour déplacer la caméra le long de la canule ; et **caractérisé par**
un capteur de position (50), qui est fixé à une paroi interne de l'extrémité distale de la canule et est configuré pour générer des signaux indiquant la position de l'extrémité distale dans l'organe, le câblage du capteur (59), qui fournit des données de localisation du capteur de position, passant du capteur de position à travers l'élément mobile.

2. Trocart selon la revendication 1, l'élément mobile étant configuré pour être glissé dans un canal (70) formé sur la paroi interne d'une canule, parallèlement à l'axe longitudinal, et une extrémité distale de l'élément mobile étant couplée à la caméra.

3. Trocart selon la revendication 2, et comprenant une poignée de commande de glissement (60) couplée à une extrémité proximale de l'élément mobile, la poignée de commande de glissement comprenant un bouton de commande de position de caméra rotatif (66) configuré pour être tourné et, en étant tourné, pour déplacer l'élément mobile et la caméra.

4. Trocart selon n'importe quelle revendication précédente, le capteur de position étant un capteur de position magnétique.

5. Trocart selon n'importe quelle revendication précédente, la caméra étant inclinée par rapport à l'axe longitudinal, de manière à avoir une direction de vision qui capture une ouverture distale (78) de la canule.

6. Appareil (28) comprenant :
le trocart selon la revendication 1 ; et
une poignée de commande à glissière couplée à une extrémité proximale de l'élément mobile et configurée pour déplacer l'élément mobile et la caméra.

7. Appareil selon la revendication 6, l'élément mobile étant configuré pour être glissé dans un canal formé sur une paroi interne d'une canule, parallèlement à l'axe longitudinal, une extrémité distale de l'élément mobile étant couplée à la caméra, et la poignée de commande de glissement comprenant un bouton de commande de position de caméra rotatif configuré pour être tourné et, en étant tourné, faire glisser l'élément mobile.

8. Système comprenant :
le trocart selon l'une quelconque des revendications 1 à 5 ; et
un processeur qui est configuré pour :
à l'aide des signaux générés par le capteur de position, estimer la position de l'extrémité distale du trocart dans l'organe.

9. Système selon la revendication 8, le processeur étant en outre configuré pour :
sur la base de la position estimée, enregistrer une image acquise par la caméra avec une image médicale de référence ; et
présenter l'image acquise par la caméra et l'image médicale de référence, enregistrées l'une par rapport à l'autre, à un utilisateur.
